# EUROPÄISCHE PATENTSCHRIFT

(11) **EP 2 808 052 B1**
(45) Veröffentlichungstag und Bekanntmachung des Hinweises auf die Patenterteilung: **22.02.2017**
(21) Anmeldenummer: 14164764.4
(22) Anmeldetag: 15.04.2014
(51) Int. Cl.: A61M 39/10, A61M 39/00

(54) **Kupplungsvorrichtung zum Verbinden von Schläuchen, insbesondere zahnmedizinischer Versorgungsschläuche**
Coupling device for connecting hoses, in particular dental supply hoses
Dispositif de couplage de tuyaux, en particulier de tuyaux d'alimentation dentaires

(30) Priorität: 31.05.2013 DE 102013105611
(43) Veröffentlichungstag der Anmeldung: 03.12.2014
(73) Patentinhaber: MEDTRONIC medizinisch-elektronische Geräte-Gesellschaft mit beschränkter Haftung, 61276 Weilrod (DE)
(72) Erfinder: Schmidt, Alfred, 61250 Usingen (DE)
(74) Vertreter: Glück Kritzenberger Patentanwälte PartGmbB

(56) Entgegenhaltungen:
- WO-A1-2013/036854
- WO-A1-2013/050319
- WO-A2-02/090813
- GB-A- 2 175 968
- US-A- 3 667 785
- US-A- 6 059 325
- US-A1- 2013 158 343
- US-A1- 2013 164 706

## Beschreibung

Die Erfindung betrifft eine Kupplungsvorrichtung zum Verbinden von Schläuchen, insbesondere zahnmedizinischer Versorgungsschläuche gemäß dem Oberbegriff des Patentanspruches 1.

Für den Betrieb zahnärztlicher Instrumente, insbesondere elektrisch und/oder pneumatisch betriebener zahnärztlicher Instrumente ist es notwendig, die Instrumente mit Versorgungs- und/oder Steuereinheiten zu verbinden. Die Verbindung der Instrumente mit den Versorgungs- und Steuereinheiten erfolgt dabei über zahnmedizinische Versorgungsschläuche. Dazu sind Kupplungsvorrichtungen bekannt, die einerseits das jeweilige zahnärztliche Instrument mit einem Versorgungsschlauch als auch den Versorgungsschlauch mit einer Versorgungs- und Steuereinheit verbinden können.

Ein zahnärztliches Instrument besteht üblicherweise aus einem Handstückteil und einem darin aufgenommenen Behandlungswerkzeug, beispielsweise in Form eines Bohrers oder dergleichen. Auch wird unter einem zahnärztlichen Instrument ein vorzugsweise in Ultraschalltechnologie realisierter Zahnsteinentferner oder ein Spülinstrument verstanden. Genannte zahnärztliche Instrumente weisen in Ihrem jeweiligen Handstückteil elektrisch oder pneumatisch betriebene Motoreinheiten oder Ultraschalleinheiten auf, welche mittels des Versorgungsschlauches von der Versorgungs- und Steuereinheit mit den zum Betrieb erforderlichen Fluiden, elektrischer Energie und/oder elektrischen Steuersignalen versorgt werden.

Bei der Versorgung und Steuerung zahnmedizinischer Instrumente kann auch eine Verbindung zwischen zwei Versorgungsschläuchen oder zwischen Schlauchstücken erforderlich sein. Zum Verbinden zweier Versorgungsschläuche oder Schlauchstücke werden häufig Kupplungsvorrichtungen verwendet, die Kupplungen mittels Kegeln oder Gewinden oder so genannte Luer-Lock-Kupplungen ermöglichen. Bei derart bekannten Kupplungsvorrichtungen kann der Kupplungszustand jedoch nur hergestellt bzw. wieder gelöst werden, wenn die Kupplungsvorrichtung gut zugänglich und mit den Fingern umgreifbar ist. Das Abdrehen des einen Kupplungsteils vom anderen führt dabei auch zu einem Verdrehen oder Verdrillen der mit den Kupplungsteilen verbundenen Schlauchstücken, was in vielen Fällen sehr nachteilig ist.

Die DE 196 37 266 C1 beschreibt eine Kupplung zur Verbindung eines Schlauches mit einem medizinischen Instrument oder mit einem anderen Schlauch, bei der im gekuppelten Zustand ein erstes Kupplungsteil mit einer konvexen Kugelfläche an einer konkaven Kugelfläche eines zweiten Kupplungsteils anliegt, wobei beide Kupplungsteile dichtend axial gegeneinander verspannt sind. Dazu ist das erste Kupplungsteil in eine am zweiten Kupplungsteil befindliche Aufnahme einschwenkbar und zum Lösen des Kupplungszustandes wieder ausschwenkbar.

Zwar erfordert das Herstellen und Lösen des gekuppelten Zustandes der in der DE 196 37 266 C1 offenbarten Kupplung kein Abdrehen des einen Kupplungsteils vom anderen und behebt so die vorgenannten Nachteile, jedoch ist das Einschwenken des ersten Kupplungsteils in das zweite Kupplungsteil bzw. das Ausschwenken lediglich in einer einzigen Richtung möglich, da die am zweiten Kupplungsteil befindliche Aufnahme eine starre Begrenzung bzw. einen Anschlag darstellt. In US 3667785 ist das Ausschwenken nur mit Hilfe von O-Ringen möglich.

Aufgabe der Erfindung ist es daher, eine Kupplungsvorrichtung zum Verbindung von Schläuchen, insbesondere zahnmedizinischer Versorgungsschläuche bereitzustellen, welche das Herstellen und Lösen eines Kupplungszustandes auf besonders einfache und für die Schläuche bzw. Versorgungsschläuche schonende Weise auch unter räumlich beengten Bedingungen ermöglicht. Die Aufgabe wird ausgehend von den Merkmalen des Oberbegriffes des Patentanspruches 1 durch dessen kennzeichnende Merkmale gelöst.

Die Erfindung betrifft eine Kupplungsvorrichtung zum Verbinden von Schläuchen, insbesondere zahnmedizinischer Versorgungsschläuche bestehend aus zumindest einem mit einem ersten Schlauchstück verbindbaren männlichen Verbindungsteil mit einem männlichen Eingriffsabschnitt und wenigstens einem mit einem zweiten Schlauchstück verbindbaren und lösbar mit dem männlichen Verbindungsteil kuppelbaren weiblichen Verbindungsteil mit einem weiblichen Eingriffsabschnitt.

Der wesentliche Aspekt der vorliegenden Erfindung ist darin zu sehen, dass der männliche Eingriffsabschnitt zumindest eine Ringrippe mit einer gewölbten Anlagefläche und der weibliche Eingriffsabschnitt zumindest einen Wölbungsabschnitt aufweist. Im Kupplungszustand kommt es zu einem Hintergreifen der Ringrippe durch den Wölbungsabschnitt des weiblichen Eingriffsabschnittes und der Wölbungsabschnitt liegt unter Ausbildung einer haltenden Verbindung an der gewölbten Anlagefläche der Ringrippe des männlichen Eingriffsabschnittes an. Aufgrund eines Verkippens oder Verschwenkens des männlichen Verbindungsteils relativ zum weiblichen Verbindungsteil ist der Kupplungszustand durch ein Abgleiten der gewölbten Anlagefläche der Ringrippe vom Wölbungsabschnitt lösbar.

Das weibliche und männliche Verbindungsteil der erfindungsgemäßen Kupplungsvorrichtung zum Verbinden von Schläuchen, insbesondere zahnmedizinischer Versorgungsschläuche weisen je eine Längsachse und einen im Wesentlichen kreisrunden Querschnitt sowie eine zentrale Durchgangsbohrung auf. Im Kupplungszustand sind die Verbindungsteile so angeordnet, dass die Längsachsen der beiden Verbindungsteile in einer gemeinsamen Hauptachse zusammenfallen und die Durchgangsbohrungen fluchtend zueinander ausgerichtet sind. Dadurch wird sicher gestellt, dass der Transport bzw. der Fluss von Fluiden ungehindert durch die Kupplungsvorrichtung stattfinden kann. In besonders bevorzugten Ausführungsformen kann in der Kupplungsvorrichtung auch ein Ventil zum gerichteten und kontrollierten Fluss von Fluiden integriert sein.

Die erfindungsgemäße Kupplungsvorrichtung, welche auch als Minischnellkupplung bezeichnet werden kann, weist geringe Außenmaße auf und eignet sich daher auch zur Verwendung unter räumlich beengten Bedingungen.

Besonders vorteilhaft kann ein erstes Schlauchstück über die erfindungsgemäße Kupplungsvorrichtung in besonders einfacher Weise mit einem zweiten Schlauchstück verbunden bzw. gekoppelt werden. Das erste Schlauchstück ist dabei mit dem männlichen Verbindungsteil der Kupplungsvorrichtung und das zweite Schlauchstück mit dem weiblichen Verbindungsteil der Kupplungsvorrichtung verbunden. Durch einfaches Zusammenstecken kann der Kupplungszustand hergestellt werden, indem beide Verbindungsteile in koaxialer Ausrichtung gekuppelt werden. In diesem Kupplungszustand greifen der weibliche und männliche Eingriffsabschnitt derart ineinander, dass der Wölbungsabschnitt des weiblichen Verbindungsteiles die Ringrippe des männlichen Verbindungsteiles hintergreift. Eine haltende Verbindung zwischen beiden Verbindungsteilen wird dadurch hergestellt, dass der Wölbungsabschnitt im weiblichen Eingriffsabschnitt an der gewölbten Anlagefläche der Ringrippe im männlichen Eingriffsabschnitt anliegt. Die axiale Krafteinwirkung der haltenden Verbindung im Kupplungszustand erfolgt gleichmäßig verteilt über den Umfang der Verbindungsteile, so dass im Kupplungszustand in der koaxialen Ausrichtung der Verbindungsteile ein ausreichender und sicherer Halt gewährleistet ist.

Ganz besonders vorteilhaft kann der Kupplungszustand der Kupplungsvorrichtung durch Verkippen oder Verschwenken der beiden Verbindungsteile zueinander in besonders einfacher und schneller Weise gelöst werden. Durch das Verkippen ist die axiale Krafteinwirkung nicht mehr gleichmäßig verteilt über den gesamten Umfang der Verbindungsteile. An der Stelle, an der die durch das Verkippen auftretende axiale Zugkraft am größten ist, kommt es in der erfindungsgemäßen Kupplungsvorrichtung aufgrund der gewölbten Ausbildung des Wölbungsabschnittes und der gewölbten Anlagefläche zu einem Abgleiten des Wölbungsabschnittes von der gewölbten Anlagefläche der Ringrippe. Der Kupplungszustand wird gelöst und die Verbindungsteile und somit die Schlauchstücke liegen wieder getrennt vor.

Ganz besondere Vorteile bringt die erfindungsgemäße Kupplungsvorrichtung mit sich, da zum Verkippen oder Verschwenken der Verbindungsteile gegeneinander keine vorgegebene Schwenk- oder Kipprichtung eingehalten werden muss, sondern das Verschwenken vielmehr in jeder Richtung möglich ist. Zudem reicht bei der erfindungsgemäßen Kupplungsvorrichtung ein kleine Schwenkbewegung aus, um den Kupplungszustand zu lösen, das heißt, die beiden Verbindungsteile müssen lediglich in eine Stellung zueinander gebracht werden, in der die beiden Längsachsen einen stumpfen Winkel einschließen, der nur geringfügig kleiner als 180° ist. Vor allem im Hinblick darauf, dass Verbindungen zwischen Versorgungsschläuchen oder Schlauchstücken unter sehr beengten räumlichen Verhältnissen, nämlich beispielsweise innerhalb eines Gehäuses einer Versorgungseinheit gelöst werden sollen, ist dies von besonderer Bedeutung.

Bevorzugt weist der weibliche Eingriffsabschnitt einen an eine Eingriffsöffnung anschließenden konischen Öffnungsabschnitt und einen an den konischen Öffnungsabschnitt anschließenden ersten zylindrischen Ringabschnitt auf. Ebenso bevorzugt ist der Wölbungsabschnitt zwischen dem an den konischen Öffnungsabschnitt anschließenden ersten zylindrischen Ringabschnitt und einem zweiten zylindrischen Ringabschnitt angeordnet. Der konische Öffnungsabschnitt, welcher die lichte Öffnungsweite ausgehend von der Eingriffsöffnung hin zum ersten zylindrischen Ringabschnitt reduziert, erleichtert das Einführen des männlichen Verbindungsteils. Die lichte Öffnungsweite und damit der Innendurchmesser des ersten zylindrischen Ringabschnittes wird mittels des Wölbungsabschnittes in Richtung des zweiten zylindrischen Ringabschnittes erweitert, so dass der zweite zylindrische Ringabschnitt einen größeren Innendurchmesser aufweist als der erste zylindrische Ringabschnitt. Durch die Anordnung des Wölbungsabschnittes zwischen dem ersten und dem zweiten zylindrischen Ringabschnitt liegt ein konvex gewölbter Übergang zwischen den zylindrischen Ringabschnitten vor, eine kantige Stufung kann dadurch vermieden werden.

Die Anordnung des Wölbungsabschnittes zwischen dem ersten und dem zweiten zylindrischen Ringabschnitt führt im Kupplungszustand zu einem Hintergreifen der Ringrippe des männlichen Verbindungsteils durch den ersten zylindrischen Ringabschnitt. Dieses Hintergreifen der Ringrippe kann auch als eine Art Einrasten des ersten zylindrischen Ringabschnittes hinter der Ringrippe verstanden werden. Das Hintergreifen bzw. Einrasten erfolgt aufgrund des im Vergleich zum Außendurchmesser der Ringrippe verkleinerten Innendurchmessers des ersten zylindrischen Ringabschnittes. Der zweite zylindrische Ringabschnitt mit einem Innendurchmesser, der in etwa dem am Scheitelpunkt der Ringrippe gemessenen Außendurchmesser der Ringrippe entspricht, liegt im Kupplungszustand dem Scheitelpunkt der Ringrippe an. Durch das beschriebene gegenseitige Ineinandergreifen bzw. durch das Anliegen oder Anlagern der genannten Abschnitte des männlichen und weiblichen Eingriffsabschnittes wird eine dichtende Verbindung zwischen den beiden Verbindungsteilen hergestellt.

Gemäß einer bevorzugten Ausführungsform der vorliegenden Erfindung weist der weibliche Eingriffsabschnitt zusätzlich einen zylindrischen Basisabschnitt auf, wobei der zylindrische Basisabschnitt unter Ausbildung einer Schulter an den zweiten zylindrischen Ringabschnitt anschließt. Der Innendurchmesser des zylindrischen Basisabschnittes ist dabei größer als diejenige des zweiten zylindrischen Ringabschnittes.

Besondere Vorteile ergeben sich dadurch dass der männliche Eingriffsabschnitt einen gerundeten Nippelendabschnitt aufweist und dass die Ringrippe auf der dem Nippelendabschnitt zugewandten Seite eine Einführwölbung zum erleichterten gleitenden Einführen in den weiblichen Eingriffsabschnitt aufweist. Die Einführwölbung der Ringrippe liegt der gewölbten Anlagefläche gegenüber und weist bevorzugt einen Krümmungsradius in einem Bereich von 4 mm bis 6 mm auf. Besonders bevorzugt weist die gewölbte Anlagefläche der Ringrippe einen Krümmungsradius in einem Bereich von 2 mm bis 4 mm auf.

Vorteilhaft weist das männliche Verbindungsteil im Bereich des männlichen Eingriffsabschnittes zusätzlich eine umlaufende Ringnute zur Aufnahme eines Dichtungselementes auf, wobei die Ringnute auf der dem Nippelendabschnitt abgewandten Seite an die Ringrippe anschließt. Durch ein in die Ringnute eingelegtes Dichtungselement kann die Kupplungsvorrichtung zusätzlich abgedichtet werden, so dass für die zu verbindenden Versorgungsschläuche sicher gestellt ist, dass beim Transport von Fluiden keine Leckstellen innerhalb der Kupplungsvorrichtung auftreten. Als Dichtungselemente können beispielsweise Dichtungsringe aus Gummi oder Kunststoff, insbesondere O-Ringe verwendet werden.

Bevorzugt weist der Wölbungsabschnitt des weiblichen Eingriffsabschnittes eine konvexe Wölbung mit einem Krümmungsradius in einem Bereich von 1 mm bis 3 mm auf.

Gemäß einer besonders bevorzugten Ausführungsform der vorliegenden Erfindung sind zur Verbindung des männlichen Verbindungsteils mit einem ersten Schlauchstück und zur Verbindung des weiblichen Verbindungsteils mit einem zweiten Schlauchstück gerade Schlauchverbinder vorgesehen. Beispielsweise können die Schlauchverbinder aus Kunststoff oder aus Metall, insbesondere aus Messing hergestellt sein.

Besonders bevorzugt sind das männliche und weibliche Verbindungsteil aus einem Kunststoff, insbesondere aus einem thermoplastischen Kunststoff, beispielsweise aus Polypropylen hergestellt.

Die Erfindung soll nachfolgend anhand von Ausführungsbeispielen im Zusammenhang mit den Figuren näher erläutert werden. Zudem ergeben sich Weiterbildungen, Vorteile und Anwendungsmöglichkeiten der Erfindung auch aus der nachfolgenden Beschreibung der Ausführungsbeispiele und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Es wird aber ausdrücklich darauf hingewiesen, dass die Erfindung keinesfalls auf die angegebenen Beispiele beschränkt sein soll. Es zeigen
- Fig. 1: schematisch dargestellt eine Kupplungsvorrichtung gemäß der vorliegenden Erfindung in seitlicher Ansicht,
- Fig. 2: eine Längsschnittdarstellung eines männlichen Verbindungsteils,
- Fig. 3: eine Längsschnittdarstellung eines weiblichen Verbindungsteils,
- Fig. 4: schematisch dargestellt einen Längsschnitt durch die Kupplungsvorrichtung der Figur 1 und
- Fig. 5: den Ausschnitt A der Figur 4 in vergrößerter Darstellung.

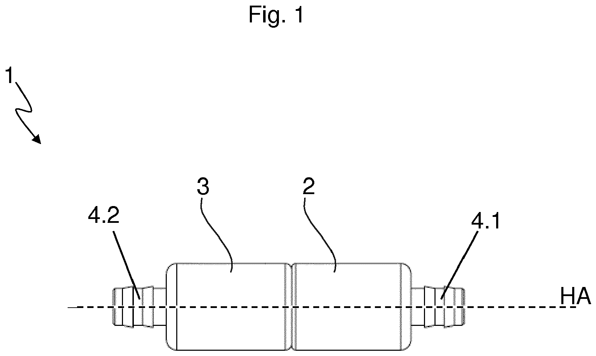

In der Figur 1 ist eine seitliche Ansicht einer neuerungsgemäßen Kupplungsvorrichtung 1 zum Verbinden von Schläuchen, insbesondere zahnmedizinischer Versorgungsschläuche im Kupplungszustand dargestellt.

Die Kupplungsvorrichtung 1 besteht aus einem mit einem ersten Schlauchstück verbindbaren männlichen Verbindungsteil 2 und einem mit einem zweiten Schlauchstück verbindbaren und lösbar mit dem männlichen Verbindungsteil 2 kuppelbaren weiblichen Verbindungsteil 3. Im dargestellten Beispiel sind zur Verbindung der Schlauchstücke mit den beiden Verbindungsteilen 2, 3 zwei gerade Schlauchverbinder 4.1, 4.2 vorgesehen. Bei den Schlauchverbindern 4.1, 4.2 handelt es sich beispielsweise um einfache Steckverbinder, welche an den den Kupplungsseiten abgewandten Enden des männlichen und weiblichen Verbindungsteils 2,3 eingesteckt sind und zum Aufstecken von Schlauchstücken ausgebildet sind.

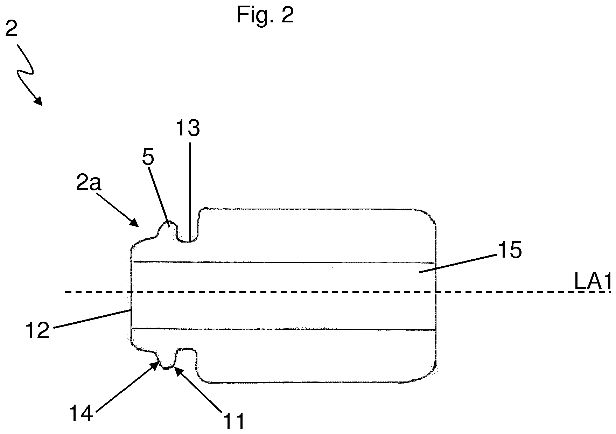

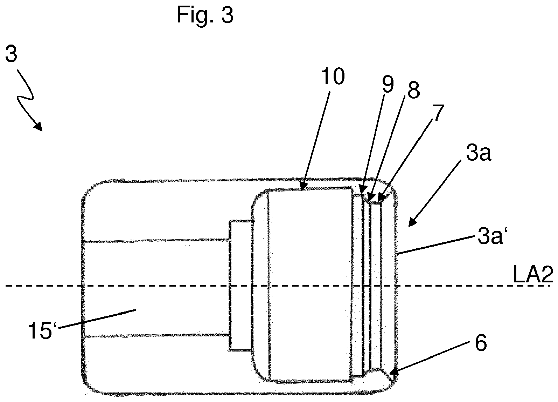

Das weibliche und männliche Verbindungsteil 2, 3 der erfindungsgemäßen Kupplungsvorrichtung 1 zum Verbinden von Schläuchen, insbesondere zahnmedizinischer Versorgungsschläuche weisen einen im Wesentlichen kreisrunden Querschnitt mit einem jeweiligen Außendurchmesser in einem Bereich von 6 mm bis 7 mm auf. Im Kupplungszustand sind die Verbindungsteile so angeordnet, dass Längsachsen LA1, LA2 (in der Figur 1 nicht dargestellt, siehe Figuren 2 und 3) der beiden Verbindungsteile 2, 3 in einer gemeinsamen Hauptachse HA zusammenfallen. Beide Verbindungsteile 2,3 sowie die Schlauchverbinder 4.1, 4.2 sind in dem in Figur 1 dargestellten Kupplungszustand koaxial angeordnet.

In den Figuren 2 und 3 sind jeweils ein männliches Verbindungsteil 2 bzw. ein weibliches Verbindungsteil 3 in einem Längsschnitt dargestellt. Das in Figur 2 dargestellte männliche Verbindungsteil 2 weist eine entlang einer Längsachse LA1 verlaufende zentrale Durchgangsbohrung 15 für den Fluidtransport und einen männlichen Eingriffsabschnitt 2a zum Eingriff in das weibliche Verbindungsteil 3 auf. Der männliche Eingriffsabschnitt 2a mit einem abgerundeten Nippelendabschnitt 12 liegt in axialer Richtung dem mit einem Schlauchstück verbindbaren Ende des männlichen Verbindungsteils 2 gegenüber. Erfindungsgemäß weist das männliche Verbindungsteil 2 im Bereich des männlichen Eingriffsabschnittes 2a eine Ringrippe 5 auf. Die umlaufend ausgebildete Ringrippe 5 schließt in axialer Richtung an den Nippelendabschnitt 12 an und stellt auf der dem Nippelendabschnitt 12 zugewandten Seite eine Einführwölbung 14 zum erleichterten Einführen bzw. Einschieben in das weibliche Verbindungsteil 3 zur Verfügung. Die umlaufende Ringrippe 5 weist auf der der Einführwölbung 14 gegenüberliegenden Seite eine gewölbte Anlagefläche 11 auf, wobei die gewölbte Anlagefläche 11 zur Ausbildung einer im Kupplungszustand haltenden, aber lösbaren Anlage am weiblichen Verbindungsteil 3 ausgebildet ist.

Der am Scheitelpunkt der Ringrippe 5 gemessene Außendurchmesser der Ringrippe 5 ist etwa 1,2fach bis 1,4fach größer als der Außendurchmesser des Nippelendabschnittes 12, welcher in einem Bereich von rund 4 mm bis 4,5 mm liegt.

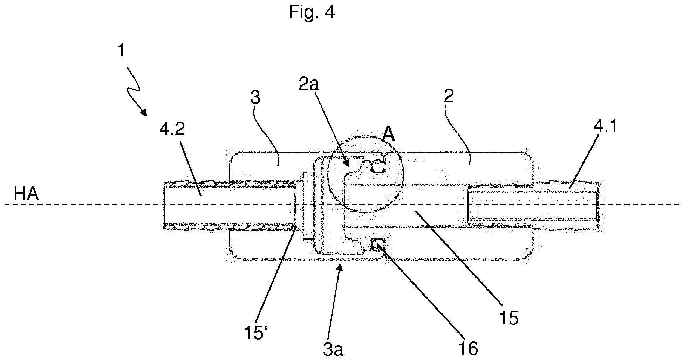

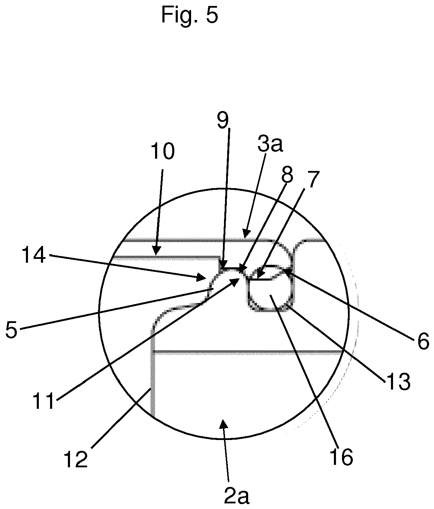

Im dargestellten Beispiel schließt sich der Ringrippe 5 auf der dem Nippelendabschnitt 12 abgewandten Seite eine umlaufende Ringnute 13 zur Aufnahme eines Dichtungselementes 16 (in der Figur 2 nicht dargestellt, siehe Figuren 4 und 5) an.

Das in Figur 3 dargestellte weibliche Verbindungsteil 3 weist ebenfalls eine entlang einer Längsachse LA2 verlaufende zentrale Durchgangsbohrung 15' und einen weiblichen Eingriffsabschnitt 3a für den Eingriff des männlichen Verbindungsteils 2 auf. Der weibliche Eingriffsabschnitt 3a mit seiner Eingriffsöffnung 3a' liegt in axialer Richtung dem mit einem Schlauchstück verbindbaren Ende des weiblichen Verbindungsteils 3 gegenüber. Die Eingriffsöffnung 3a' weist eine lichte Öffnungsweite in einem Bereich von 5 mm bis 5,8 mm auf. Erfindungsgemäß weist das weibliche Verbindungsteil 3 im Bereich des weiblichen Eingriffsabschnittes 3a zumindest einen Wölbungsabschnitt 8 auf, welcher im Kupplungszustand unter Ausbildung einer haltenden Verbindung der gewölbten Anlagefläche 11 der Ringrippe 5 des männlichen Verbindungsteils 2 anliegt.

Im dargestellten Beispiel ist der Wölbungsabschnitt 8 zwischen einem ersten zylindrischen Ringabschnitt 7 und einem zweiten zylindrischen Ringabschnitt 9 angeordnet, wobei der Innendurchmesser des ersten zylindrischen Ringabschnittes 7 mit rund 4,9 mm bis 5,3 mm kleiner ist als der Innendurchmesser des zweiten zylindrischen Ringabschnittes 9. Zur Eingriffsöffnung 3a' hin schließt sich dem ersten zylindrischen Ringabschnitt 7 ein konischer Öffnungsabschnitt 6 an, welcher ein erleichtertes Einführen des männlichen Verbindungsteils 2 in das weibliche Verbindungsteil 3 sicher stellt. Auf der der Eingriffsöffnung 3a' abgewandten Seite schließt sich an den zweiten zylindrischen Ringabschnitt 9 ein zylindrischer Basisabschnitt 10 an, in welchem im Kupplungszustand der Nippelendabschnitt 12 des männlichen Verbindungsteils 2 aufgenommen ist.

Ausgehend von der Eingriffsöffnung 3a' des weiblichen Eingriffsabschnittes 3a schließen somit in axialer Richtung der Reihe nach der konische Öffnungsabschnitt 6, der erste zylindrische Ringabschnitt 7, der Wölbungsabschnitt 8, der zweite zylindrische Ringabschnitt 9 und der zylindrischer Basisabschnitt 10 aneinander an. Diese Aufeinanderfolge der genannten Abschnitte des weiblichen Eingriffsabschnittes 3a in Kombination mit der wie oben beschriebenen Ausbildung des männlichen Eingriffsabschnittes 2a ermöglicht sowohl ein einfaches Verbinden wie auch ein einfaches Trennen des männlichen und weiblichen Verbindungsteils 2, 3.

Die Figur 4 zeigt schematisch einen Längsschnitt durch die Kupplungsvorrichtung 1 im Kupplungszustand. Der männliche Eingriffsabschnitt 2a des männlichen Verbindungsteils 2 ist im Kupplungszustand vom weiblichen Eingriffsabschnitt 3a des weiblichen Verbindungsteils 3 aufgenommen. Das männliche und weibliche Verbindungsteil 2, 3 sind dadurch koaxial entlang der gemeinsamen Hauptachse HA angeordnet, wodurch die zentralen Durchgangsbohrungen 15, 15' fluchtend zueinander ausgerichtet sind und einen ungehinderten Passageweg für den Transport bzw. den Fluss von Fluiden zur Verfügung stellen. Im Kupplungszustand wird eine haltende Verbindung zwischen beiden Verbindungsteilen 2, 3 dadurch hergestellt, dass der Wölbungsabschnitt 8 des weiblichen Verbindungsteils 3 an der gewölbten Anlagefläche 11 des männlichen Verbindungsteils anliegt (siehe Figur 5), wobei die axiale Krafteinwirkung der haltenden Verbindung im Kupplungszustand gleichmäßig verteilt über den Umfang beider Verbindungsteile 2, 3 erfolgt. Im Kupplungszustand ist somit in der koaxialen Ausrichtung der Verbindungsteile 2, 3 ein ausreichender und sicherer Halt gewährleistet.

In der Figur 5 ist der Ausschnitt A der Figur 4 vergrößert dargestellt und zeigt ausschnittsweise die im Kupplungszustand ineinandergreifenden männlichen und weiblichen Eingriffsabschnitte 2a, 3a. Die haltende Verbindung zwischen beiden Verbindungsteilen 2, 3 wird im Kupplungszustand dadurch hergestellt, dass der Wölbungsabschnitt 8 des weiblichen Eingriffsabschnittes 3a an der gewölbten Anlagefläche 11 der Ringrippe 5 des männlichen Eingriffsabschnittes 2a anliegt und gleichzeitig der erste zylindrische Ringabschnitt 7 die Ringrippe 5 auf der dem Nippelendabschnitt 12 abgewandten Seite hintergreift. Das Hintergreifen der Ringrippe 5 durch den ersten zylindrischen Ringabschnitt 7 kann auch als eine Art Einrasten des ersten zylindrischen Ringabschnittes 7 hinter der Ringrippe 5 verstanden werden. Das Hintergreifen bzw. Einrasten erfolgt aufgrund des im Vergleich zum Außendurchmesser der Ringrippe 5 verkleinerten Innendurchmessers des ersten zylindrischen Ringabschnittes 7. Der zweite zylindrische Ringabschnitt 9 mit einem Innendurchmesser, der etwa dem am Scheitelpunkt der Ringrippe 5 gemessenen Außendurchmesser der Ringrippe 5 entspricht, liegt dem Scheitelpunkt der Ringrippe 5 an. Durch das beschriebene gegenseitige Ineinandergreifen des männlichen und weiblichen Eingriffsabschnittes 2a, 3a, nämlich durch die Anlage des Wölbungsabschnittes 8 an der gewölbten Anlagefläche 11 der Ringrippe 5 sowie durch die Anlage des zweiten zylindrischen Ringabschnittes 9 am Scheitel der Ringrippe 5 wird mittels der Kupplungsvorrichtung eine dichtende Verbindung hergestellt. Zur Unterstützung der Abdichtung der dichtenden Verbindung weist der männliche Eingriffsabschnitt 2a im dargestellten Beispiel zusätzlich eine Ringnute 13 auf, in welcher ein Dichtungselement 16 aufgenommen ist.

Zum Lösen des Kupplungszustandes bzw. zum Trennen der Verbindung des männlichen und weiblichen Verbindungsteils 2, 3 ist lediglich ein Verkippen oder Verschwenken der beiden Verbindungsteile 2, 3 notwendig. Durch das Verkippen oder Verschwenken des männlichen Verbindungsteils 2 relativ zum weiblichen Verbindungsteil 3 ist die axiale Krafteinwirkung nicht mehr gleichmäßig verteilt über den gesamten Umfang der beiden Verbindungsteile 2, 3. An der Stelle, an der die durch das Verkippen auftretende axiale Zugkraft am größten ist, kommt es in der erfindungsgemäßen Kupplungsvorrichtung 1 zu einem Abgleiten des konvexen Wölbungsabschnittes 8 von der gewölbten Anlagefläche 11 der Ringrippe 5 und der Kupplungszustand wird gelöst. Im dargestellten Beispiel weist die gewölbte Anlagefläche 11 der Ringrippe 5 einen Krümmungsradius in einem Bereich von 2 mm bis 4 mm und der konvexe Wölbungsabschnitt 8 im weiblichen Verbindungsteil 3 einen Krümmungsradius in einem Bereich von 1 mm bis 3 mm auf.

Die Erfindung wurde voranstehend an einem Ausführungsbeispiel beschrieben. Es versteht sich, dass zahlreiche Modifikationen und Änderungen der Erfindung möglich sind im Bereich der Ansprüche.

### Bezugszeichenliste

- 1: Kupplungsvorrichtung
- 2: männliches Verbindungsteil
- 2a: männlicher Eingriffsabschnitt
- 3: weibliches Verbindungsteil
- 3a: weiblicher Eingriffsabschnitt
- 3a': Eingriffsöffnung
- 4.1, 4.2: Schlauchverbinder
- 5: Ringrippe
- 6: konischer Öffnungsabschnitt
- 7: erster zylindrischer Ringabschnitt
- 8: Wölbungsabschnitt
- 9: zweiter zylindrischer Ringabschnitt
- 10: zylindrischer Basisabschnitt
- 11: gewölbte Anlagefläche
- 12: Nippelendabschnitt
- 13: Ringnute
- 14: Einführwölbung
- 15, 15': zentrale Durchgangsbohrung
- 16: Dichtungselement

- LA1: Längsachse
- LA2: Längsachse
- HA: Hauptachse

## Patentansprüche

1. Kupplungsvorrichtung (1) zum Verbinden von Schläuchen, insbesondere zahnmedizinischer Versorgungsschläuche bestehend aus zumindest einem mit einem ersten Schlauchstück verbindbaren männlichen Verbindungsteil (2) mit einem männlichen Eingriffsabschnitt (2a) und wenigstens einem mit einem zweiten Schlauchstück verbindbaren und lösbar mit dem männlichen Verbindungsteil (2) kuppelbaren weiblichen Verbindungsteil (3) mit einem weiblichen Eingriffsabschnitt (3a), wobei der männliche Eingriffsabschnitt (2a) zumindest eine Ringrippe (5) mit einer gewölbten Anlagefläche (11) aufweist und der weibliche Eingriffsabschnitt (3a) zumindest einen Wölbungsabschnitt (8) aufweist, **dadurch gekennzeichnet, dass** im Kupplungszustand der Wölbungsabschnitt (8) des weiblichen Eingriffsabschnittes (3a) die Ringrippe (5) des männlichen Eingriffsabschnittes (2a) hintergreift und unter Ausbildung einer haltenden Verbindung an der gewölbten Anlagefläche (11) der Ringrippe (5) anliegt, wobei der weibliche Eingriffsabschnitt (3a) einen an eine Eingriffsöffnung (3a') anschließenden konischen Öffnungsabschnitt (6) und einen an den konischen Öffnungsabschnitt (6) anschließenden ersten zylindrischen Ringabschnitt (7) aufweist und wobei der Wölbungsabschnitt (8) eine konvexe Wölbung aufweist und zwischen dem ersten zylindrischen Ringabschnitt (7) und einem zweiten zylindrischen Ringabschnitt (9) angeordnet ist und wobei aufgrund eines Verkippens oder Verschwenkens des männlichen Verbindungsteils (2) relativ zum weiblichen Verbindungsteil (3) der Kupplungszustand durch ein Abgleiten der gewölbten Anlagefläche (11) der Ringrippe (5) vom Wölbungsabschnitt (8) lösbar ist.

2. Kupplungsvorrichtung (1) nach Anspruch 1, **dadurch gekennzeichnet, dass** der weibliche Eingriffsabschnitt (3a) zusätzlich einen zylindrischen Basisabschnitt (10) aufweist, wobei der zylindrische Basisabschnitt (10) unter Ausbildung einer Schulter an den zweiten zylindrischen Ringabschnitt (9) anschließt.

3. Kupplungsvorrichtung (1) nach Anspruch 1 oder 2, **dadurch gekennzeichnet, dass** der männliche Eingriffsabschnitt (2a) einen gerundeten Nippelendabschnitt (12) aufweist.

4. Kupplungsvorrichtung (1) nach einem der Ansprüche 1 bis 3, **dadurch gekennzeichnet, dass** die Ringrippe (5) auf der dem Nippelendabschnitt (12) zugewandten Seite eine Einführwölbung (14) zum gleitenden Einführen in den weiblichen Eingriffsabschnitt (3a) aufweist, wobei die Einführwölbung (14) der gewölbten Anlagefläche (11) der Ringrippe (5) gegenüberliegt und einen Krümmungsradius in einem Bereich von 4 mm bis 6 mm aufweist.

5. Kupplungsvorrichtung (1) nach einem der vorhergehenden Ansprüche, **dadurch gekennzeichnet, dass** die gewölbte Anlagefläche (11) der Ringrippe (5) einen Krümmungsradius in einem Bereich von 2 mm bis 4 mm aufweist.

6. Kupplungsvorrichtung (1) nach einem der vorhergehenden Ansprüche, **dadurch gekennzeichnet, dass** das männliche Verbindungsteil (2) im Bereich des männlichen Eingriffsabschnittes (2a) zusätzlich eine umlaufende Ringnute (13) zur Aufnahme eines Dichtungselementes (16) aufweist, wobei die Ringnute (13) auf der dem Nippelendabschnitt (12) abgewandten Seite an die Ringrippe (5) anschließt.

7. Kupplungsvorrichtung (1) nach einem der vorhergehenden Ansprüche, **dadurch gekennzeichnet, dass** der Wölbungsabschnitt (8) des weiblichen Eingriffsabschnittes (3a) eine konvexe Wölbung mit einem Krümmungsradius in einem Bereich von 1 mm bis 3 mm aufweist.

8. Kupplungsvorrichtung (1) nach einem der vorhergehenden Ansprüche, **dadurch gekennzeichnet, dass** zur Verbindung des männlichen Verbindungsteils (2) mit einem ersten Schlauchstück und zur Verbindung des weiblichen Verbindungsteils (3) mit einem zweiten Schlauchstück gerade Schlauchverbinder (4.1, 4.2) vorgesehen sind.

9. Kupplungsvorrichtung (1) nach einem der vorhergehenden Ansprüche, **dadurch gekennzeichnet, dass** das männliche und weibliche Verbindungsteil (2, 3) aus einem Kunststoff, insbesondere aus einem thermoplastischen Kunststoff, beispielsweise aus Polypropylen hergestellt ist.

## Claims

1. Coupling device (1) for connecting hoses, particularly dental supply hoses, consisting of at least one male connector part (2) that has a male engagement section (2a) and can be connected to a first hose section and at least one female connector part (3) that has a female engagement section (3a) and can be connected to a second hose section, and that can detachably be coupled to the male connector part (2), wherein the male engagement section (2a) comprises at least one annular rib (5) having a curved contact surface (11) and the female engagement section (3a) comprises at least one curvature section (8), **characterized in that** the curvature section (8) of the female engagement section (3a) engages in the coupled state behind the annular rib (5) of the male engagement section (2a) and abuts against the curved contact surface (11) of the annular rib (5) such that a retentive connection is formed, wherein the female engagement section (3a) comprises a conical opening section (6) adjacent to an engagement opening (3a') and a first cylindrical ring section (7) adjacent to the conical opening section (6), wherein the curvature section (8) has a convex curvature and is arranged between the first cylindrical ring section (7) and a second cylindrical ring section (9), and wherein the coupled state can be disengaged by tilting or pivoting the male connector part (2) relative to the female connector part (3) and thereby causing the curved contact surface (11) of the annular rib (5) to slide off the curvature section (8).

2. Coupling device (1) according to claim 1, **characterized in that** the female engagement section (3a) additionally comprises a cylindrical base section (10), wherein the cylindrical base section (10) adjoins the second cylindrical ring section (9) such that a shoulder is formed.

3. Coupling device (1) according to claim 1 or 2, **characterized in that** the male engagement section (2a) comprises a rounded nipple end section (12).

4. Coupling device (1) according to one of claims 1 to 3, **characterized in that** the annular rib (5) comprises on the side facing the nipple end section (12) an insertion curvature (14) for the sliding insertion into the female engagement section (3a), wherein the insertion curvature (14) lies opposite of the curved contact surface (11) of the annular rib (5) and has a curvature radius in the range of 4 mm to 6 mm.

5. Coupling device (1) according to one of the preceding claims, **characterized in that** the curved contact surface (11) of the annular rib (5) has a curvature radius in the range of 2 mm to 4 mm.

6. Coupling device (1) according to one of the preceding claims, **characterized in that** the male connector part (2) additionally features a circumferential annular groove (13) for accommodating a sealing element (16) in the region of the male engagement section (2a), wherein the annular groove (13) is arranged adjacent to the annular rib (5) on the side facing away from the nipple end section (12).

7. Coupling device (1) according to one of the preceding claims, **characterized in that** the curvature section (8) of the female engagement section (3a) has a convex curvature with a curvature radius in the range of 1 mm to 3 mm.

8. Coupling device (1) according to one of the preceding claims, **characterized in that** straight hose connectors (4.1, 4.2) are provided for connecting the male connector part (2) to a first hose section and for connecting the female connector part (3) to a second hose section.

9. Coupling device (1) according to one of the preceding claims, **characterized in that** the male and the female connector part (2, 3) are made of plastic, particularly a thermoplastic polymer such as polypropylene.

## Revendications

1. Dispositif d'accouplement (1) destiné à assembler des flexibles, notamment des flexibles d'alimentation dans la médecine dentaire, constitué d'au moins une pièce d'assemblage (2) mâle susceptible d'être assemblée avec un premier tronçon de flexible, avec une portion d'engagement (2a) mâle et au moins une pièce d'assemblage (3) femelle, susceptible d'être assemblée avec un deuxième tronçon de flexible et qui peut être détachablement accouplée avec la pièce d'assemblage (2) mâle, avec une portion d'engagement (3a) femelle, la portion d'engagement (2a) mâle comportant au moins une nervure annulaire (5) avec une surface d'appui (11) voûtée et la portion d'engagement (3a) femelle comportant au moins une portion voûtée (8),
**caractérisé en ce qu'**en position accouplée, la portion voûtée (8) de la portion d'engagement (3a) femelle s'accroche par l'arrière dans la nervure annulaire (5) de la portion d'engagement (2a) mâle et en formant un assemblage de retenue, s'appuie sur la surface d'appui (11) voûtée de la nervure annulaire (5), la portion d'engagement (3a) femelle comportant une portion d'ouverture (6) conique se raccordant sur une ouverture d'engagement (3a') et une première portion annulaire (7) cylindrique se raccordant sur la portion d'ouverture (6) conique et la portion voûtée (8) comportant une voussure convexe et étant placée entre la première portion annulaire (7) cylindrique et une deuxième portion annulaire (9) cylindrique et suite à un basculement ou pivotement de la pièce d'assemblage (2) mâle par rapport à la pièce d'assemblage (3) femelle, la position d'accouplement étant amovible par un retrait glissant de la surface d'appui (11) voûtée de la nervure annulaire (5) de la portion voutée (8).

2. Dispositif d'accouplement (1) selon la revendication 1, **caractérisé en ce que** la portion d'engagement (3a) femelle comporte en supplément une portion de base (10) cylindrique, la portion de base (10) cylindrique se raccordant sur la deuxième portion annulaire (9) cylindrique, en formant un épaulement.

3. Dispositif d'accouplement (1) selon la revendication 1 ou 2, **caractérisé en ce que** la portion d'engagement (2a) mâle comporte une portion d'extrémité en mamelon (12) arrondie.

4. Dispositif d'accouplement (1) selon l'une quelconque des revendications 1 à 3, **caractérisé en ce que** sur le côté qui fait face à la portion d'extrémité en mamelon (12), la nervure annulaire (5) comporte une voussure d'introduction (14) pour l'introduction par glissement dans la portion d'engagement (3a) femelle, la voussure d'introduction (14) étant opposée à la surface d'appui (11) voûtée de la nervure annulaire (5) et présentant un rayon de courbure dans un ordre de 4 mm à 6 mm.

5. Dispositif d'accouplement (1) selon l'une quelconque des revendications précédentes, **caractérisé en ce que** la surface d'appui (11) voûtée de la nervure annulaire (5) présente un rayon de courbure dans un ordre de 2 mm à 4 mm.

6. Dispositif d'accouplement (1) selon l'une quelconque des revendications précédentes, **caractérisé en ce que** dans la zone de la portion d'engagement (2a) mâle, la pièce d'assemblage (2) mâle comporte en supplément une rainure annulaire (13) périphérique destinée à recevoir un élément d'étanchéité (16), sur le côté opposé à la portion d'extrémité en mamelon (12), la rainure annulaire (13) se raccordant sur la nervure annulaire (5).

7. Dispositif d'accouplement (1) selon l'une quelconque des revendications précédentes, **caractérisé en ce que** la portion voûtée (8) de la portion d'engagement (3a) femelle présente une voussure convexe, avec un rayon de courbure dans l'ordre de 1 mm à 3 mm.

8. Dispositif d'accouplement (1) selon l'une quelconque des revendications précédentes, **caractérisé en ce que** des connecteurs de flexibles (4.1, 4.2) droits sont prévus pour assembler la pièce d'assemblage (2) mâle avec un premier tronçon de flexible et pour assembler la pièce d'assemblage (3) femelle avec un deuxième tronçon de flexible.

9. Dispositif d'accouplement (1) selon l'une quelconque des revendications précédentes, **caractérisé en ce que** la pièce d'assemblage (2, 3) mâle et femelle est fabriquée en une matière plastique, notamment en une matière thermoplastique, par exemple en polypropylène.
